# EUROPEAN PATENT APPLICATION

(11) **EP 4 809 914 A2**
(43) Date of publication of application: **23.09.2026**
(21) Application number: 26158141.7
(22) Date of filing: 12.02.2026
(51) Int. Cl.: A61B 5/055, A61B 5/00, G16H 30/40, G16H 50/20

(54) **ABDOMINAL IMAGE-BASED ANALYSIS SYSTEM AND METHOD THEREOF**

(30) Priority: 18.03.2025 TW 114110156
(71) Applicant: ASUSTek Computer Inc., Peitou, Taipei-City 112 (TW)
(72) Inventor: CHEN, Yu-Cheng, 112 Taipei City (TW); LIOU, Fou-Ming, 112 Taipei City (TW); SHEN, Yu-Jie, 112 Taipei City (TW); DENG, Chih-Ying, 112 Taipei City (TW); CHEN, Tzu-An, 112 Taipei City (TW)
(74) Representative: Viering, Jentschura & Partner mbB Patent- und Rechtsanwälte

(57) **Abstract**

An abdominal image-based analysis system (10) and a method thereof are provided. The analysis system (10) includes a computing device (12) and a display device (16). In the analysis system (10), a regression deep learning model (14) is implemented into the computing device (12). The computing device (12) receives at least one abdominal image (18) and inputs the abdominal image (18) into the regression deep learning model (14). The regression deep learning model (14) extracts a physiological characteristic value in the abdominal image (18) and executes an analysis and regression operation to generate a predicted physiological index (20). The display device (16) is electrically connected to the computing device (12) to display the abdominal image (18) and the corresponding predicted physiological index (20). Therefore, the abdominal image (18) is combined with artificial intelligence (AI) technology to generate an objective predictive physiological index (20) to assist doctors in analysis.

## Description

### CROSS-REFERENCE TO RELATED APPLICATION

This application claims the priority benefit of Taiwan application serial No. 114110156, filed on March 18, 2025. The entirety of the above-mentioned patent application is hereby incorporated by reference herein and made a part of specification.

### BACKGROUND OF THE INVENTION

### Field of the Invention

The invention relates to an abdominal image-based analysis system combined with artificial intelligence technology and an analysis method thereof.

### Description of the Related Art

Abdominal Computed Tomography (CT) is a non-invasive diagnostic imaging examination. The abdominal CT utilizes a high-dose of special X-rays to take images of an internal body from a plurality of angles. In combination with advanced computer technology, clear three-dimensional images of cross-sections of the body are formed to display conditions of internal organs to help detect diseases of internal organs in abdomen. Magnetic Resonance Imaging (MRI) utilizes a powerful magnetic field and an electromagnetic effect under the magnetic field for imaging to provide clear images of tissue structures of the abdomen. MRI has advantages of a high degree of tissue contrast resolution and multi-directional scanning capabilities without any radiation. Then, MRI provides the most accurate and reliable images of the tissue structure inside a human body for diagnosis of lesions and a proposal of treatment measures.

Whether the abdominal computed tomography method or the abdominal magnetic resonance imaging method is used, a doctor usually carefully examines and makes a diagnosis by directly referring to information such as abdominal CT scan images or abdominal MRI images after an abdominal image is obtained. However, it is timewasting, has low efficient, and is rather inconvenient by manually and visually checking abnormalities in the abdominal image.

### BRIEF SUMMARY OF THE INVENTION

An abdominal image-based analysis system is provided. The abdominal image-based analysis system comprises: a computing device, implementing with a regression deep learning model, the computing device receives at least one abdominal image and inputs the abdominal image to the regression deep learning model, the regression deep learning model extracts a physiological characteristic value of the abdominal image and executes an analysis and regression operation to generate a predicted physiological index; and a display device electrically connected to the computing device to display the abdominal image and the corresponding predicted physiological index.

An analysis method based on an abdominal image is provided. The analysis method comprises: obtaining at least one abdominal image; inputting the abdominal image to a regression deep learning model, the regression deep learning model extracts a physiological characteristic value of the abdominal image and executes an analysis and regression operation to generate a predicted physiological index; and displaying the abdominal image and the corresponding predicted physiological index.

In conclusion, according to an abdominal image-based analysis system and a method there of in embodiments, after an abdominal image is obtained, the abdominal image is combined with artificial intelligence (AI) technology to generate an objective predictive physiological index. The objective predictive physiological index assists doctors in analysis. Moreover, the accuracy and efficiency of diagnosis are improved, conditions of patients are better and more quickly understood by doctors, and thus a more effective treatment plan is formulated. Therefore, objective and easily understandable data is provided to avoid medical disputes. Furthermore, an abdominal image-based analysis system and a method thereof in embodiments are more convenient, and results are quickly displayed during a diagnosis and treatment process.

### BRIEF DESCRIPTION OF THE DRAWINGS

FIG. 1 is a block diagram of an analysis system according to an embodiment of the invention.
FIG. 2 is a schematic diagram showing an architecture for inference and prediction via an analysis system according to an embodiment of the invention.
FIG. 3 is a block diagram of an analysis system according to an embodiment of the invention.
FIG. 4 is a block diagram of an analysis system according to an embodiment of the invention.
FIG. 5 is a schematic diagram showing an architecture for an analysis system applying an abdominal computed tomography (CT) scan image to a regression deep learning model of sarcopenia for inference and prediction according to an embodiment of the invention.
FIG. 6 is a schematic diagram showing an architecture for an analysis system applying an abdominal computed tomography (CT) scan image to a regression deep learning model of fatty liver for inference and prediction according to an embodiment of the invention.
FIG. 7 is a schematic diagram showing an architecture for an analysis system applying an abdominal computed tomography (CT) scan image to a regression deep learning model of abdominal visceral fat volume for inference and prediction according to an embodiment of the invention.
FIG. 8 is a block diagram showing a regression deep learning model used by a computing device in an analysis system according to an embodiment.

### DETAILED DESCRIPTION OF THE EMBODIMENTS

Embodiments of the invention are disclosed in the following drawings, and for more clarity, the details of the disclosure will be described hereinafter. According to the following description and the claims, the advantages and features of this application will be clearer. It should be noted that the drawings are in a very simplified form and all use imprecise proportions, which are only used to conveniently and clearly assist in explaining the purpose of the embodiments.

An abdominal image-based analysis system and a method thereof in embodiments are based on an abdominal computed tomography (CT) image or an abdominal magnetic resonance imaging (MRI) image combined with a regression deep learning model to extract a physiological characteristic value in the image, execute an analysis and regression operation, and provide a predicted physiological index (value), such as the index for sarcopenia, fatty liver, body composition, to doctors as important reference information in auxiliary diagnosis.

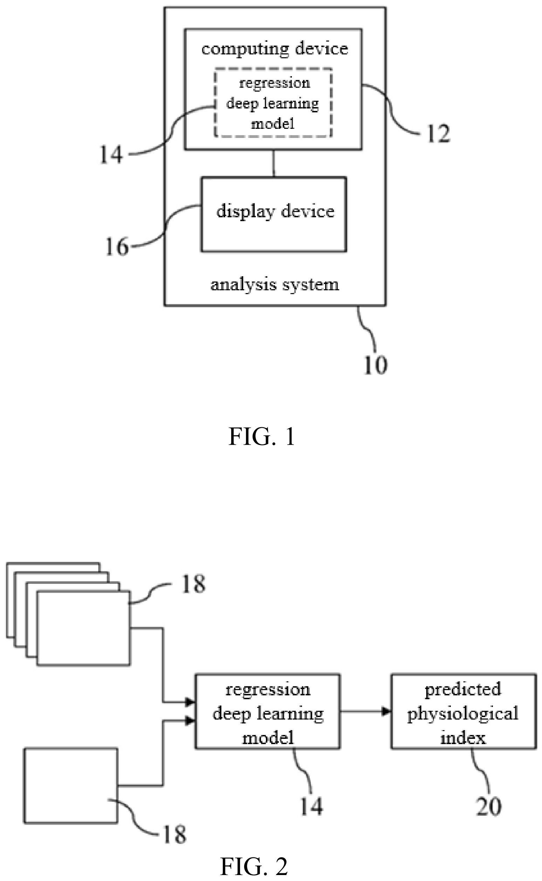

Please refer to FIG. 1 and FIG. 2. An abdominal image-based analysis system 10 includes a computing device 12 and a display device 16. A regression deep learning model 14 is implemented in the computing device 12. After the computing device 12 receives at least one abdominal image 18, the computing device 12 inputs the abdominal image 18 to the regression deep learning model 14. The regression deep learning model 14 extracts the physiological characteristic value of the abdominal image and executes an analysis and regression operation to generate a predicted physiological index 20. In an embodiment, the abdominal image 18 is an abdominal CT scan image or an abdominal MRI image. The display device 16 is electrically connected to the computing device 12 to receive the abdominal image 18 from the computing device 12 and the corresponding predicted physiological index 20, and the abdominal image 18 and the predicted physiological index 20 are displayed on the display device 16 to provide a reference basis for subsequent diagnosis to doctors.

Please refer to FIG. 1 and FIG. 2. In an embodiment, after the computing device 12 obtains an abdominal video 18 or a plurality of abdominal images 18, the computing device 12 inputs the abdominal video 18 or the abdominal images 18 to the regression deep learning model 14. The regression deep learning model 14 is a 3D regression deep learning model. The regression deep learning model 14 extracts the physiological characteristic value of the abdominal image 18 and executes an analysis and regression operation to generate the final predicted physiological index 20. The abdominal image 18 and the corresponding predicted physiological index 20 are displayed on the display device 16.

Please refer to FIG. 1 and FIG. 2. In an embodiment, after the computing device 12 obtains the abdominal image 18, the computing device 12 inputs the abdominal image 18 to the regression deep learning model 14. The regression deep learning model 14 is a 2D regression deep learning model. The regression deep learning model 14 extracts the physiological characteristic value of the abdominal image 18 and executes the analysis and regression operation to generate the final predicted physiological index 20. Then, the abdominal image 18 and the corresponding predicted physiological index 20 are displayed on the display device 16.

In an embodiment, the computing device 12 is an electronic device capable of executing independent calculations, such as a computer, and the computing device 12 cooperates with the display device 16, which is not limited herein. In an embodiment, a notebook replaces the computing device 12 and the display device 16, and the notebook is simultaneously responsible for operations of the computing device 12 and the display device 16.

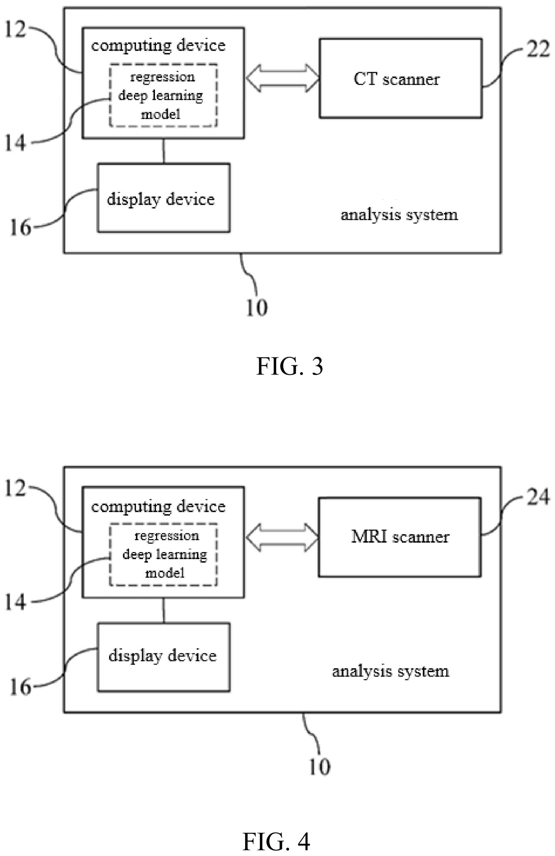

Please refer to FIG. 2 and FIG. 3. In an embodiment, the analysis system 10 further comprising a CT scanner 22 connected to the computing device 12. The CT scanner 22 is used to take pictures of a subject to obtain one or a plurality of abdominal CT scan images as the abdominal image 18. As a result, after the computing device 12 receives the abdominal CT scan image, the predicted physiological index 20 is quickly and objectively generated directly via the regression deep learning model 14 and provided to doctors as a reference for auxiliary diagnosis.

Please refer to FIG. 2 and FIG. 4. In an embodiment, the analysis system 10 further includes an MRI scanner 24 electrically connected to the computing device 12. The MRI 24 scanner is used to take pictures of the subject to obtain one or a plurality of abdominal MRI images as the abdominal image 18. After the computing device 12 receives the abdominal MRI image, the predicted physiological index 20 is quickly and objectively generated directly via the regression deep learning model 14 and provided to doctors as a reference for auxiliary diagnosis.

Please refer to FIG. 1 and FIG. 2. The regression deep learning model 14 in the computing device 12 is an independent artificial intelligence (AI) model and a trained deep learning model. Consequently, before the regression deep learning model 14 is used, corresponding models are implemented and trained for different applications.

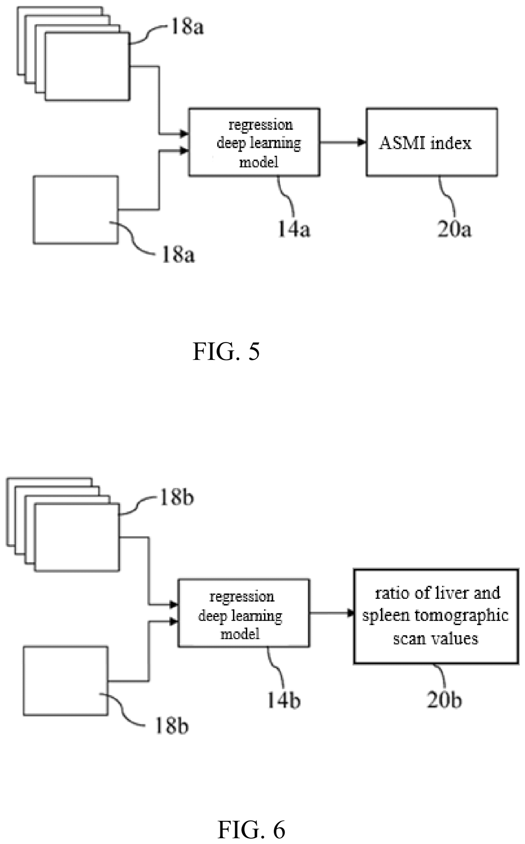

Please refer to FIG. 1 and FIG. 5. In an embodiment, the abdominal CT scan image 18a is used to a regression deep learning model 14a for the sarcopenia. Before the regression deep learning model 14a is used for computational prediction, the computing device 12 is used to train the regression deep learning model 14a. During a training process, the abdominal CT scan image and a corresponding appendicular skeletal muscle mass index (ASMI) are first obtained. The abdominal CT scan image is used as input data for training, and the corresponding ASMI index is used as label data. Then, the AI model to be trained is trained based on the input data and the label data. The AI model to be trained executes the analysis and regression operation on feature values of the input image for training to obtain the trained regression deep learning model 14a. The trained regression deep learning model 14a is adapted to make inferences about the sarcopenia. When the regression deep learning model 14a is used to make inference and prediction about the sarcopenia, the computing device 12 inputs the abdominal CT scan image 18a into the regression deep learning model 14a to generate an ASMI index 20a as the predicted physiological index 20. The predicted physiological index 20 is used as a reference basis in the diagnosis of the sarcopenia.

Please refer to FIG. 1 and FIG. 6. In an embodiment, the abdominal CT scan image 18b is used to a regression deep learning model 14b for the fatty liver. Before the regression deep learning model 14b is used for computational prediction, the computing device 12 is used to train the regression deep learning model 14b. During the training process, the abdominal CT scan image and corresponding average tomographic scan values of the liver and the spleen are obtained, and a ratio of liver and spleen tomographic scan values is obtained. The abdominal CT scan image is used as input data for training, and the corresponding ratio of liver and spleen tomographic scan values is used as the label data. Then, the AI model to be trained is trained based on the input data and the label data. The AI model to be trained executes the analysis and regression operation on feature values of the input image for training to obtain the trained regression deep learning model 14b. The trained regression deep learning model 14b is adapted to be used to make inferences about fatty liver. When the regression deep learning model 14b is used to make inference and prediction about fatty liver, the computing device 12 inputs the abdominal CT scan image 18b into the trained regression deep learning model 14b to generate a ratio 20b of the liver and spleen tomographic scan values as the predicted physiological index 20. The predicted physiological index 20 is used as a reference basis in the diagnosis of fatty liver.

In an embodiment, for the regression deep learning model 14a applied to the sarcopenia and the regression deep learning model 14b applied to fatty liver, the regression deep learning models 14a and 14b are 3D regression deep learning models correspondingly when the abdominal CT scan images 18a and 18b are a video or a plurality of images respectively. The regression deep learning models 14a and 14b are 2D regression deep learning models correspondingly when the abdominal CT scan image 18a and the abdominal CT scan image 18b are one image, respectively.

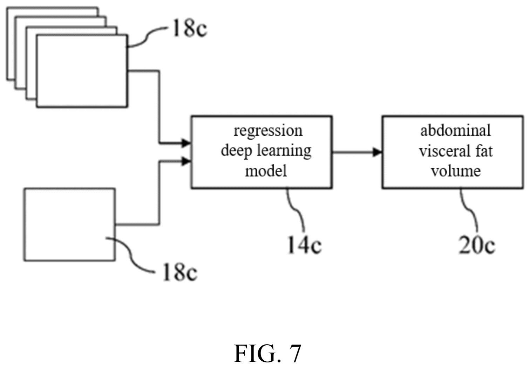

Please refer to FIG. 1 and FIG. 7. In an embodiment, an abdominal MRI image 18c is used to a regression deep learning model 14c for abdominal visceral fat volume. Before the regression deep learning model 14c is used for computational prediction, the computing device 12 is used to train the regression deep learning model 14c. During the training process, the abdominal MRI image and a corresponding abdominal visceral fat volume are obtained. The abdominal MRI image is used as input data for training, and the corresponding abdominal visceral fat volume is used as the label data. Then, the AI model to be trained is trained based on the input data and the label data. The AI model to be trained executes the analysis and regression operation on feature values of the input image for training to obtain the trained regression deep learning model 14c. The trained regression deep learning model 14c is adapted to be used to make inferences about the abdominal visceral fat volume. When the regression deep learning model 14c is used to make inference and prediction about the abdominal visceral fat volume 20c, the computing device 12 inputs the abdominal MRI image 18c into the trained regression deep learning model 14c to generate the abdominal visceral fat volume 20c as the predicted physiological index 20. The predicted physiological index 20 is used as a reference basis in the diagnosis of the abdominal visceral fat volume.

In an embodiment, for the regression deep learning model 14a applied to the sarcopenia and the regression deep learning model 14b applied to fatty liver, when the aforementioned abdominal CT scan images 18a and 18b are a video or a plurality of images, respectively, the regression deep learning models 14a and 14b are 3D regression deep learning models correspondingly. When the abdominal CT scan image 18a and the abdominal CT scan image 18b are one image, respectively, the regression deep learning models 14a and 14b are 2D regression deep learning models correspondingly.

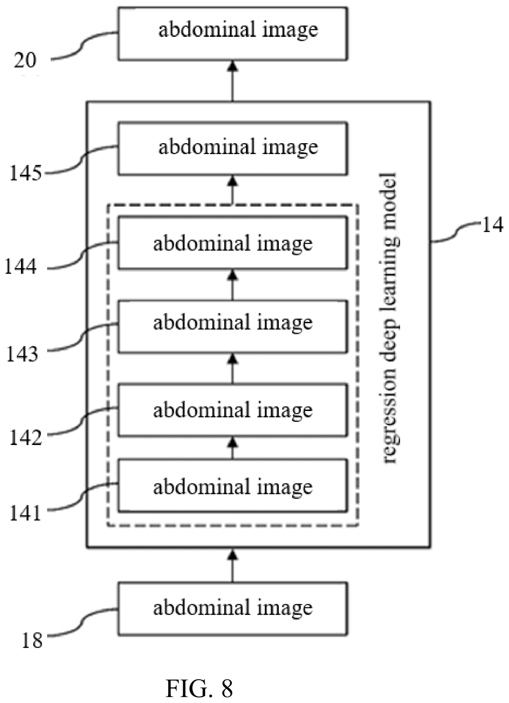

Please refer to FIG. 1 and FIG. 8. In an embodiment, the regression deep learning model 14 receives the abdominal image 18 and executes feature extraction and regression analysis operations on the abdominal image 18 to output the predicted physiological index 20 corresponding to the abdominal image 18. The regression deep learning model 14 includes a convolutional layer 141, a batch normalization layer 142, an activation function layer 143, a normalization layer 144 and a fully connected layer 145. The convolutional layer 141 is connected to the batch normalization layer 142. The batch normalization layer 142 is connected to the activation function layer 143. The activation function layer 143 is connected to the normalization layer 144. The normalization layer 144 is connected to the fully connected layer 145. The convolutional layer 141 executes a convolutional operation. Then, after a normalization process of the batch normalization layer 142, an activation operation of the activation function layer 143, a regularization process of the normalization layer 144, and a regression operation process of the fully connected layer 145, the predicted physiological index 20 is output.

In conclusion, according to an abdominal image-based analysis system and a method provided in the disclosure, the abdominal image is combined with artificial intelligence (AI) technology to generate an objective predictive physiological index after an abdominal image is obtained. The objective predictive physiological index assists doctors in analysis. Moreover, the accuracy and efficiency of diagnosis are improved, conditions of patients are better and more quickly understood by doctors, and thus a more effective treatment plan is formulated. Therefore, objective and easily understandable data is provided to avoid medical disputes. Furthermore, an abdominal image-based analysis system and a method thereof in embodiments are more convenient, and results are quickly displayed during a diagnosis and treatment process.

Although the present invention has been described in considerable detail with reference to certain preferred embodiments thereof, the disclosure is not for limiting the scope of the invention. Persons having ordinary skill in the art may make various modifications and changes without departing from the scope. Therefore, the scope of the appended claims should not be limited to the description of the preferred embodiments described above.

## Claims

1. An abdominal image-based analysis system (10), comprising:
a computing device (12), implementing with a regression deep learning model (14), the computing device (12) receives at least one abdominal image (18) and inputs the abdominal image (18) to the regression deep learning model (14), the regression deep learning model (14) extracts a physiological characteristic value of the abdominal image (18) and executes an analysis and regression operation to generate a predicted physiological index (20); and
a display device (16) electrically connected to the computing device (12) to display the abdominal image (18) and the corresponding predicted physiological index (20).

2. The abdominal image-based analysis system (10) according to claim 1, wherein the abdominal image (18) is an abdominal computed tomography (CT) scan image (18a, 18b) or an abdominal magnetic resonance imaging (MRI) image (18c).

3. The abdominal image-based analysis system (10) according to claim 2, wherein when the abdominal image (18) is the abdominal CT scan image (18a), the computing device (12) inputs the abdominal CT scan image (18a) into the regression deep learning model (14) to generate an appendicular skeletal muscle mass index (ASMI) (20a) as the predicted physiological index (20), and the predicted physiological index (20) is used as a reference basis in a diagnosis of sarcopenia.

4. The abdominal image-based analysis system (10) according to claim 3, wherein when the abdominal CT scan image (18a) is a video or a plurality of images, the regression deep learning model (14) is a 3D regression deep learning model; and when the abdominal CT scan image (18a) is one image, the regression deep learning model (14) is a 2D regression deep learning model.

5. The abdominal image-based analysis system (10) according to claim 2, wherein when the abdominal image (18) is the abdominal CT scan image (18b), the computing device (12) inputs the abdominal CT scan image (18b) into the regression deep learning model (14) to generate a ratio of liver and spleen tomographic scan value (20b) as the predicted physiological index (20), and the predicted physiological index (20) is used as a reference basis in a diagnosis of fatty liver.

6. The abdominal image-based analysis system (10) according to claim 5, wherein when the abdominal CT scan image (18b) is a video or a plurality of images, the regression deep learning model (14) is a 3D regression deep learning model; and when the abdominal CT scan image (18b) is an image, the regression deep learning model (14) is a 2D regression deep learning model.

7. The abdominal image-based analysis system (10) according to claim 2, wherein when the abdominal image (18) is the abdominal MRI image (18c), the computing device (12) inputs the abdominal MRI image (18c) to the regression deep learning model (14) to generate an abdominal visceral fat volume (20c) as the predicted physiological index (20), and the predicted physiological index (20) is used as a reference basis in a diagnosis of visceral fat.

8. The abdominal image-based analysis system (10) according to claim 7, wherein when the abdominal MRI image (18c) is a video or a plurality of images, the regression deep learning model (14) is a 3D regression deep learning model; and when the abdominal MRI image (18c) is an image, the regression deep learning model (14) is a 2D regression deep learning model.

9. The abdominal image-based analysis system (10) according to claim 2, further comprising a CT scanner (22) connected to the computing device (12) to generate the abdominal CT scan image (18a, 18b).

10. The abdominal image-based analysis system (10) according to claim 2, further comprising an MRI scanner (24) connected to the computing device (12) to generate the abdominal MRI image (18c).

11. An analysis method based on an abdominal image (18), comprising:
obtaining at least one abdominal image (18);
inputting the abdominal image (18) to a regression deep learning model (14), the regression deep learning model (14) extracts a physiological characteristic value of the abdominal image (18) and executes an analysis and regression operation to generate a predicted physiological index (20); and
displaying the abdominal image (18) and the corresponding predicted physiological index (20).

12. The analysis method according to claim 11, wherein the abdominal image (18) is an abdominal CT scan image (18a, 18b) or an abdominal MRI image (18c).

13. The analysis method according to claim 12, wherein when the abdominal image (18) is the abdominal CT scan image (18a), the abdominal CT scan image (18a) is input to the regression deep learning model (14) to generate an appendicular skeletal muscle mass index (ASMI) (20a) as the predicted physiological index (20), and the predicted physiological index (20) is used as a reference basis in a diagnosis of sarcopenia.

14. The analysis method according to claim 13, wherein when the abdominal CT scan image (18a) is a video or a plurality of images, the regression deep learning model (14) is a 3D regression deep learning model; and when the abdominal CT scan image (18a) is an image, the regression deep learning model (14) is a 2D regression deep learning model.

15. The analysis method according to claim 12, wherein when the abdominal image (18) is the abdominal CT scan image (18b), the computing device (12) inputs the abdominal CT scan image (18b) into the regression deep learning model (14) to generate a ratio of liver and spleen tomographic scan value (20b) as the predicted physiological index (20), and the predicted physiological index (20) is used as a reference basis in a diagnosis of fatty liver.

16. The analysis method according to claim 15, wherein when the abdominal CT scan image (18b) is a video or a plurality of images, the regression deep learning model (14) is a 3D regression deep learning model; and when the abdominal CT scan image (18b) is an image, the regression deep learning model (14) is a 2D regression deep learning model.

17. The analysis method according to claim 12, wherein when the abdominal image (18) is the abdominal MRI image (18c), the abdominal MRI image (18c) is input to the regression deep learning model (14) to generate an abdominal visceral fat volume (20c) as the predicted physiological index (20), and the predicted physiological index (20) is used as a reference basis in a diagnosis of visceral fat.

18. The analysis method according to claim 17, wherein when the abdominal MRI image (18c) is a video or a plurality of images, the regression deep learning model (14) is a 3D regression deep learning model; and when the abdominal MRI image (18c) is an image, the regression deep learning model (14) is a 2D regression deep learning model.
